# EUROPÄISCHE PATENTSCHRIFT

(11) **EP 0 825 256 B1**
(45) Veröffentlichungstag und Bekanntmachung des Hinweises auf die Patenterteilung: **25.06.2003**
(21) Anmeldenummer: 97113697.3
(22) Anmeldetag: 07.08.1997
(51) Int. Cl.: C12N 5/24, C07K 16/46

(54) **Hybridzelle und deren Verwendung zur Herstellung eines Arzneimittels zur Induktion einer Tumorimmunität**
Hybrid cell and its use for the preparation of a drug for the induction of tumourimmunity
Cellule hybride et son utilisation pour préparer un médicament pour induire une réponse immune contre une tumeur

(30) Priorität: 23.08.1996 DE 19634159
(43) Veröffentlichungstag der Anmeldung: 25.02.1998
(73) Patentinhaber: GSF-Forschungszentrum für Umwelt und Gesundheit GmbH, 85764 Oberschleissheim (DE)
(72) Erfinder: Mocikat, Ralf, 81369 München (DE); Lindhofer, Horst, 82194 Gröbenzell (DE); Thierfelder, Stefan, 82223 Eichenau (DE)
(74) Vertreter: Reinhard - Skuhra - Weise & Partner

(56) Entgegenhaltungen:
- EP-A- 0 657 533
- WO-A-92/08802
- WO-A-95/06481
- WO-A-95/16775
- WO-A-96/26964
- DE-C- 4 419 399
- DE-C- 19 634 159
- DE-C- 19 835 633
- US-A- 5 126 253
- STREHL JOHN ET AL: "Gene therapy of b-cell lymphoma with cytokine gene-modified trioma cells." INTERNATIONAL JOURNAL OF CANCER, Bd. 83, Nr. 1, 24. September 1999 (1999-09-24), Seiten 113-120, XP000972506 ISSN: 0020-7136
- MOCIKAT RALPH ET AL: "Trioma-based vaccination against B-cell lymphoma confers long-lasting tumor immunity." CANCER RESEARCH, Bd. 57, Nr. 12, 1997, Seiten 2346-2349, XP000979564 ISSN: 0008-5472
- SCHLAUDER G G ET AL: "BIOCHEMICAL CHARACTERIZATION OF I-A-K PROTEINS FROM MUTANT ANTIGEN-PRESENTING B CELL B LYMPHOMA HYBRIDOMAS" MOLECULAR IMMUNOLOGY, Bd. 22, Nr. 5, 1985, Seiten 597-607, XP000972507 ISSN: 0161-5890
- WAHL U ET AL: "Trioma cells for the therapy of human B-cell lymphoma." EUROPEAN JOURNAL OF CANCER, Bd. 35, Nr. SUPPL. 5, Oktober 1999 (1999-10), Seite S57 XP000972513 5th International Symposium on the Biological Therapy of Cancer: From Basic Research to Clinical Applications;Munich, Germany; October 27-30, 1999 ISSN: 0959-8049

## Beschreibung

Die Erfindung betrifft eine Hybridzelle und deren Verwendung zur Induktion einer Tumorimmunität durch Injektion in einen an einer B-Zell-Neoplasie erkrankten Patienten.

Maligne Erkrankungen der B-Zellen des Menschen (B-Zell-Neoplasien), beispielsweise B-Zell-Lymphome, haben trotz der Fortschritte der Chemo- und Radiotherapie der letzten Jahre noch immer eine äußerst ungünstige Prognose. Eine Heilung dieser Erkrankungen ist nicht möglich. Es ist daher notwendig, neue Behandlungsstrategien zu entwickeln. Große Hoffnungen werden dabei auf immuntherapeutische Ansätze gesetzt, mittels derer das Immunsystem des Patienten dazu gebracht werden soll, den Tumor abzustoßen.

Es ist bekannt, daß auf Tumorzellen Tumor-assoziierte Antigene vorkommen und daß das Immunsystem prinzipiell durchaus in der Lage ist, diese zu erkennen und die malignen Zellen anzugreifen. Tumoren haben jedoch verschiedene Strategien entwickelt, die es ihnen erlauben, sich der Immunantwort zu entziehen. Dies gelingt ihnen z.B. durch ungenügende Präsentation von Tumor-assoziierten Antigenen und/oder durch unzureichende Aktivierung der in der Regel vorhandenen Tumor-spezifischen T-Zellen.

Maligne B-Zellen, beispielsweise B-Zell-Lymphome, zeichnen sich dadurch aus, daß sie ein absolut tumorspezifisches Antigen besitzen, nämlich den Idiotyp des von ihnen exprimierten Immunglobulins. Dieser ist jedoch ein sehr schwaches Immunogen.

Es ist eine Aufgabe der vorliegenden Erfindung, ein neues Mittel bereitzustellen, um maligne Erkrankungen der B-Zellen des Menschen zu therapieren.

Diese Aufgabe wir erfindungsgemäß durch die im Anspruch 1 näher gekennzeichnete Hybridzelle gelöst. Bevorzugte Ausgestaltungen der Erfindung ergeben sich aus den Unteransprüchen. Die erfindungsgemäß bereitgestellte Hybridzelle wird zur Induktion einer Tumorimmunität durch Injektion der Hybridzelle in einen an einer B-Zell-Neuplasie erkrankten Patienten, aus welchem die maligne B-Zelle stammt, verwendet.

Die erfindungsgemäße Lösung dieser Aufgabe zielt darauf ab, eine effiziente T-Zell-Antwort gegen den Idiotyp zu induzieren, indem dessen Präsentation auf professionellen Antigen-präsentierenden Zellen (APC) verstärkt wird und so eine effektivere Aktivierung von T-Zellen zustandekommt.

Die Erfindung beruht auf der spezifischen Redirektion des Tumor-Idiotyps gegen professionelle APC's. Dazu werden maligne B-Zellen, beispielsweise Lymphomzellen, aus dem Patienten mit einem Hybridom fusioniert, das Antikörper gegen Oberflächenproteine auf APC's produziert. Die Hybrid-Tumorzellen werden dem Patienten zurückinfundiert. Dadurch wird folgendes erreicht:

Die Hybrid-Tumorzellen sezernieren einen bispezifischen Antikörper (bsAk), der einerseits das Tumor-spezifische Immunglobulin enthält (1 H- und 1 L-Kette) und andererseits ein Antikörper-Halbmolekül (1 H- und 1 L-Kette), welches ein Oberflächenantigen auf APC's erkennt. Aufgrund der Bindung an APC's wird der Tumor-Idiotyp internalisiert, prozessiert und in Form Tumor-spezifischer Peptide dem Immunsystem präsentiert. Durch die "professionelle" Präsentation des Tumorantigens auf der APC, im Gegensatz zu der unvollständigen Präsentation auf der Tumorzelle, können Tumor-spezifische T-Zellen generiert werden. Aufgrund des xenogenen Anteils der Hybridzelle, der aus dem Fusionspartner der malignen B-Zelle, - dem Maus- oder Rattenhybridom -, stammt, wird diese vom Immunsystem relativ schnell als fremd erkannt und zerstört.

Dies bietet zwei Vorteile, nämlich die selbständige Zerstörung dieser artifiziellen Zellen im Patienten und zweitens eine weitere Verstärkung der Tumorimmunität durch Phagocytose der lysierten Zellen mit anschließender Präsentation weiterer Tumorantigene. Das führt dazu, daß durch Injektion der Hybridzellen eine signifikant bessere Antitumor-Wirksamkeit erzielt werden kann als durch Injektion des ex vivo produzierten und gereinigten bsAk.

Nach Fusion zweier Antikörper-produzierender Zellen entsteht im allgemeinen eine Mischpopulation von bivalenten Antikörpern mit unterschiedlichen H-/L-Ketten-Paarungen. Eine wichtige Voraussetzung für die Injektion der bsAk-produzierenden Zellen in den Tumor-tragenden Wirt ist die gefundene präferentielle H/L-Paarung zwischen Ketten derselben Spezifität. Diese wird beobachtet, wenn Zellen unterschiedlicher Spezies-Herkunft miteinander fusioniert werden (Verfahren zur Herstellung von heterologen bispezifischen Antikörpern, DE-A- 44 19 399, 9. 3. 1995). Die in den Wirt zurückinjizierten Hybrid-Tumorzellen exprimieren das gewünschte bispezifische Konstrukt mit hoher Ausbeute.

Der erzielte Antitumor-Effekt wird nicht allein durch die xenogene Natur der Hybridzelle vermittelt, da eine Hybridzelle, die einen bsAK mit einem defekten APC-Bindungsarm sezerniert, keine vollständige Tumorprotektion induzieren kann. Trotzdem scheint der xenogene Anteil wichtig zu sein, indem er zusammen mit dem Mechanismus der spezifischen Idiotyp-Redirektion synergistisch wirkt. Die xenogenen Eigenschaften der Hybridzelle erlauben es, vor der Injektion auf eine Bestrahlung zu verzichten. Dies ist ein Vorteil gegenüber bisherigen Verfahren der zellulären Vakzinierung.

Es ist jedoch auch möglich, die Hybridzellen vor der Injektion durch Bestrahlung replikationsinkompetent zu machen, ohne daß dadurch die Expression des bsAk beeinträchtigt würde. Ein weiterer Vorteil der Injektion der bsAk-produzierenden Hybridzellen in den Patienten liegt im Wegfall von aufwendigen Produktions- und Reinigungsschritten zur Herstellung des bsAk in klinikrelevanten Mengen. Schließlich ist von Bedeutung, daß die malignen B-Zellen nicht an das Wachstum in Zellkultur adaptiert werden müssen, was bei menschlichen Tumoren grundsätzlich problematisch ist. Um die Zellen mit dem Hybridom zu fusionieren, ist keine Zellteilung erforderlich.

Die maligne B-Zelle, die mit der Hybridomzelle fusionert wird, kann bspw. eine B-Zell-Leukämie-Zelle, eine B-Zell-Lymphom-Zelle oder eine Plasmazytomzelle sein.

Die Hybridomzelle stammt aus der Fusion einer Lymphozytenzelle, die einen Antikörper gegen ein Oberflächenantigen auf einer APC produziert, mit einer Myelomzelle. Das Oberflächenantigen kann bspw. ein Fc-Rezeptor, ein Mannose-5-Rezeptor oder ein MHC-Klasse II-Antigen sein. Die Hybridomzelle wird durch an sich bekannte Verfahren hergestellt. Beispielsweise sei hier verwiesen auf die Erstveröffentlichung von Köhler und Milstein, Nature Band 256, 495-497 (1975) und Römpp Lexikon Biotechnologie, Georg Thime-Verlag Stuttgart, New York, 1992, Seite 379. Insbesondere können bereits existierende, auch kommerziell erhältliche Hybridomzellen verwendet werden, die die erforderlichen Merkmale aufweisen. Zum Beispiel sei hier auf die von der American Type Culture Collection erhältliche anti-Fc-Rezeptor-Hybridomzellinie 2.4G2 (ATCC HB-197) hingewiesen.

Die erfindungsgemäß bereitgestellte Hybridzelle wird in einer therapeutisch wirksamen Menge, bevorzugt zusammen mit verträglichen Träger und/oder Hilfsstoffen, wahlweise nach einer Aufreinigung an den Patienten verabreicht. Das erfindungsgemäß bereitgestellte Arzneimittel, das die erfindungsgemäße Hybridzelle in einer therapeutisch wirksamen Menge enthält, wird an den an einer B-Zell-Neoplasie erkrankten Patienten bspw. durch eine Injektion verabreicht. B-Zell-Neoplasien sind beispielsweise B-Zell-Leukämien, B-Zell-Lymphome und Plasmazytome.

Die Immunglobuline, die von der malignen B-Zelle produziert werden, assemblieren mit dem Immunglobulin der Antikörper produzierenden Hybridomzelle zu bispezifischen Antikörpern. Diese bispezifischen Antikörper werden bevorzugt an das umgebende Medium abgegeben. Dieser bispezifische Antikörper ist zur Bindung an ein Oberflächenantigen auf einer Antigen-präsentierenden Zelle befähigt, bevorzugt zur Bindung an Fc-Rezeptoren, Mannose-5-Rezeptoren und an MHC-Klasse II-Antigene.

Als Stand der Technik werden die nachfolgenden Literaturstellen genannt, die allerdings die vorliegende Erfindung weder beschreiben noch nahelegen.

Snider et al., J.Exp. Med., 1957-1963 (1990) beschreiben, daß ein Antigen (in diesem Falle Hühnereiweiß-Lysozym) auch in geringen Dosen und ohne Adjuvans stark immunogen wird, wenn es gegen Antigen-präsentierende Zellen dirigiert wird. In dieser Arbeit geschieht dies mit Hilfe bispezifischer Antikörper, die einerseits das Antigen binden und mit dem anderen Bindungsarm Oberflächenmoleküle auf der APC erkennen (Fc-Rezeptor oder MHCII).

Guo et al.beschreiben in Science 518-520, Band 263, 1994 ein Hepatom-Modell der Ratte. Die Tiere werden durch Gabe von Hybridtumor-Zellen gegen den Wildtyptumor vakziniert werden, jedoch kann nicht gegen ein molekular definiertes und spezifisches Tumorantigen immunisiert werden. Die Hybridzellen produzieren kein bispezifisches Immunglobulin, das das Tumorantigen gegen professionelle APC's dirigiert, sondern die Hybridtumor-Zelle selbst wird zur APC. Dies wird erreicht durch Fusion der Tumorzelle mit aktivierten B-Zellen.

Nachfolgend wird die Erfindung anhand eines Ausführungsbeispiels und zweier Abbildungen näher beschrieben.

### BEISPIEL:

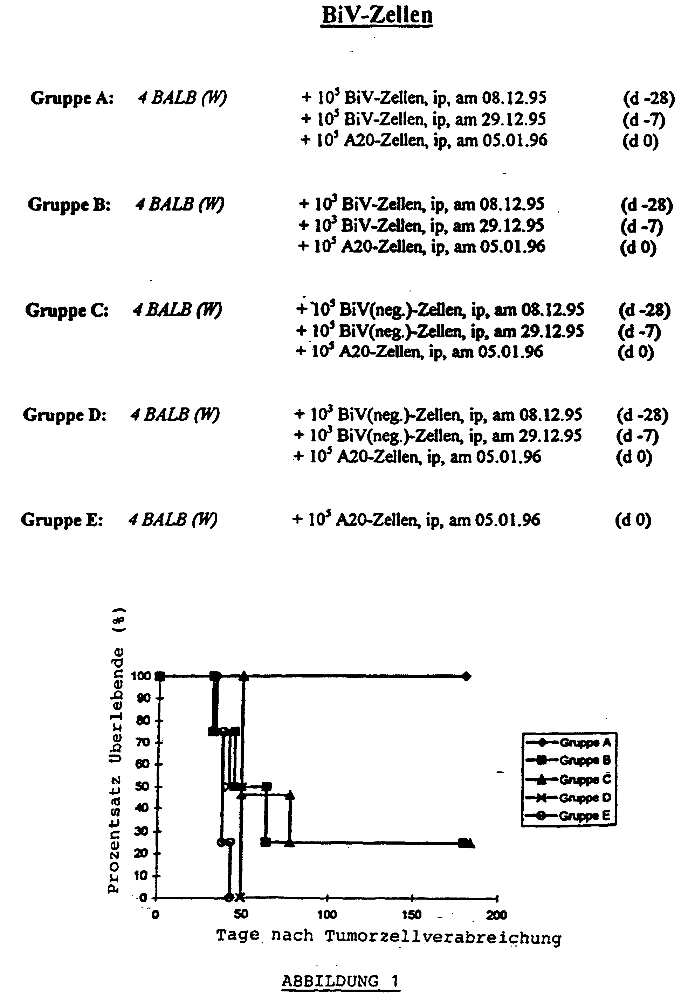

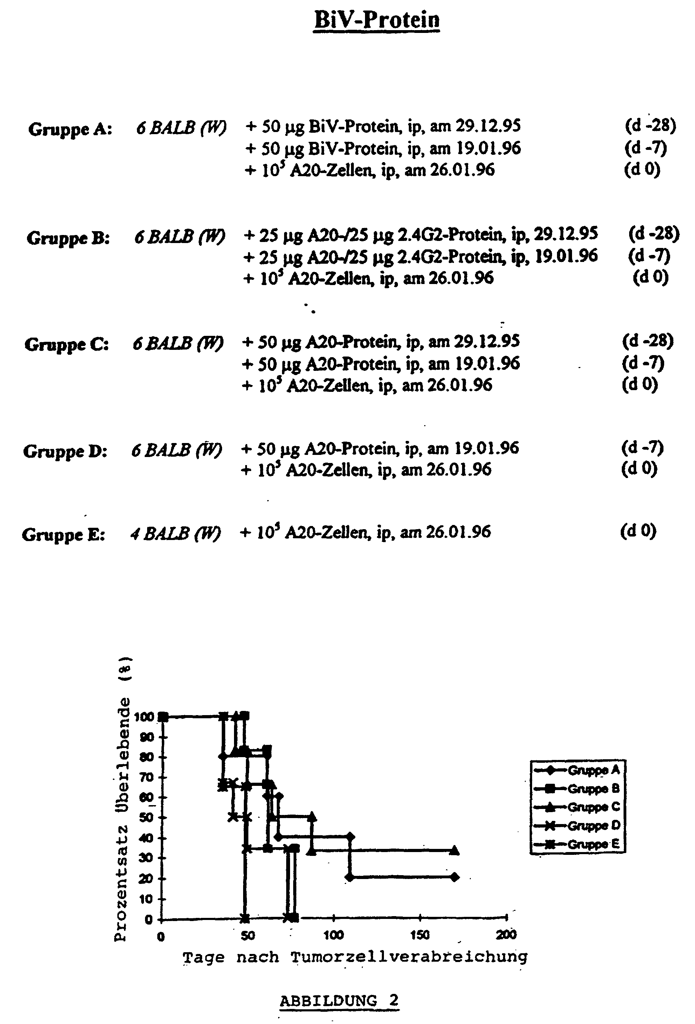

1) Das aus der BALB/c-Maus stammende B-Zell-Lymphom A20 (ATCC TIB-208) wird mit dem anti-Fc-Rezeptor-Hybridom 2.4G2 (ATCC HB-197) fusioniert. Beide Zellininen sind kommerziell bei der der American Type Culture Collection unter der angegebenen Nummer erhältlich. Dazu wird das Hybridom durch Kultur in Gegenwart von 8-Azaguanin HATsensitiv gemacht. 5 x 10⁶ Zellen des Fusionspartners werden 30 Minuten in Iodacetamid inkubiert, gewaschen und mit 1,5 x 10⁷ HAT-sensitiven Zellen gemischt. Die Fusion erfolgt durch zweiminütige Inkubation mit Polyethylenglykol 1500. Die Zellen werden in Mikrotiterplatten ausgebracht und nach zwei bis drei Tagen der Selektion in HAT-Medium zugeführt.
2) BALB/c-Mäusen werden zweimal im Abstand von 3 Wochen jeweils 10⁵ Hybridzellen (BiV-Zellen) i.p. injiziert. 7 Tage später erfolgt die i.p. Inokulation mit 10⁵ Wildtyptumor-Zellen (A20). Durch die Präimmunisierung läßt sich eine langdauernde Tumorprotektion erreichen (s. Abb. 1). Auch ein erneutes Inokulum nach über 100 Tagen wird abgestoßen. Eine Immunisierung mit 10³ Hybridzellen führt nicht zu demselben Erfolg. Hybridzellen, die einen Antikörper sezernieren, welcher nicht mehr an APC's binden kann, zeigen keine Antitumor-Wirksamkeit (BiVneg).
3) Der von BiV-Zellen sezernierte bispezifische Antikörper wird durch Chromatographie auf einer Protein A-Sepharose-Säule gereinigt. Der bsAk wird mit 0,1 M Zitratpuffer pH 5,8 von der Säule eluiert und gegen PBS dialysiert. 50 µg des gereinigten bsAk (BiV-Protein), des gereinigten monospezifischen Immunglobulins aus dem A20-Tumor bzw. eine Mischung von jeweils 25 µg dieses Immunglobulins und des anti-Fc-Rezeptor-Antikörpers werden in BALB/c-Mäuse i.p. injiziert. Nach einer weiteren Immunisierung 21 Tage später werden im Abstand von 7 Tagen 10⁵ Wildtyptumor-Zellen gespritzt (Abb. 2).

Die Erfindung wurde vorstehend anhand eines an einem Tiermodell durchgeführten Beispiels beschrieben. Die hierdurch gewonnenen Ergebnisse sind aufgrund lang dauernder Erfahrungen auf den Menschen übertragbar. Die Erfindung wurde vorstehend anhand eines speziellen Ausführungsbeispieles beschrieben. Selbstverständlich ist die Erfindung nicht auf dieses spezielle Ausführungsbeispiel beschränkt, sondern kann im Rahmen der nachfolgenden Ansprüche abgewandelt werden.

## Patentansprüche

1. Hybridzelle, herstellbar durch Fusion einer aus einem Patienten stammenden malignen B-Zelle und einer Hybridomzelle, welche einen Antikörper produziert, der an ein Oberflächenantigen auf einer Antigen-präsentierenden Zelle (APC) bindet.

2. Hybridzelle nach Anspruch 1,
**dadurch gekennzeichnet,**
**daß** die maligne B-Zelle eine B-Zell-Leukämie-Zelle, eine B-Zell-Lymphom-Zelle oder eine Plasmazytomzelle ist.

3. Hybridzelle nach Anspruch 1,
**dadurch gekennzeichnet,**
**daß** die Hybridomzelle aus der Fusion einer Lymphozytenzelle, die einen Antikörper gegen ein Oberflächenantigen auf einer APC produziert, mit einer Myelomzelle entstanden ist.

4. Hybridzelle nach einem oder mehreren der vorhergehenden Ansprüche,
**dadurch gekennzeichnet,**
**daß** die maligne B-Zelle Immunglobuline produziert, die mit dem Immunglobulin der Antikörper produzierenden Hybridomzelle zu bispezifischen Antikörpern assemblieren.

5. Hybridzelle nach einem oder mehreren der vorhergehenden Ansprüche,
**dadurch gekennzeichnet,**
**daß** der bispezifische Antikörper in das umgebende Medium sezerniert wird.

6. Hybridzelle nach einem oder mehreren der vorhergehenden Ansprüche,
**dadurch gekennzeichnet,**
**daß** der bispezifische Antikörper an Fc-Rezeptoren bindet.

7. Hybridzelle nach einem oder mehreren der vorhergehenden Ansprüche,
**dadurch gekennzeichnet,**
**daß** der bispezifische Antikörper an Mannose-5-Rezeptoren bindet.

8. Hybridzelle nach einem oder mehreren der vorhergehenden Ansprüche,
**dadurch gekennzeichnet,**
**daß** der bispezifische Antikörper an MHC-Klasse II-Antigene bindet.

9. Hybridzelle nach einem oder mehreren der vorhergehenden Ansprüche,
**dadurch gekennzeichnet,**
**daß** sie vor Injektion in den Patienten nicht replikationsinkompetent gemacht wird.

10. Hybridzelle nach einem oder mehreren der vorhergehenden Ansprüche,
**dadurch gekennzeichnet,**
**daß** sie vor Injektion in den Patienten durch Bestrahlung replikations-inkompetent gemacht wird.

11. Hybridzelle nach einem oder mehreren der vorhergehenden Ansprüche,
**dadurch gekennzeichnet, daß**
das Oberflächenantigen auf der Antigen-präsentierenden Zelle ein Fc-Rezeptor, ein Mannose-5-Rezeptor oder ein MHC-Klasse II-Antigen ist.

12. Pharmazeutische Zusammensetzung,
**dadurch gekennzeichnet, daß**
sie eine Hybridzelle nach einem oder mehreren der vorhergehenden Ansprüche in einer therapeutisch wirksamen Menge zusammen mit einem oder mehreren pharmazeutisch verträglichen Träger- und/oder Hilfsstoffen enthält.

13. Verfahren zur Herstellung einer Hybridzelle nach einem oder mehreren der vorhergehenden Ansprüche,
**dadurch gekennzeichnet, daß**
eine aus einem Patienten stammende maligne B-Zelle mit einer Hybridomzelle, welche einen Antikörper produziert, der an ein Oberflächenantigen auf einer Antigen-präsentierenden Zelle (APC) bindet, durch ein an sich bekanntes Verfahren fusioniert wird.

14. Verwendung einer Hybridzelle nach einem oder mehreren der vorhergehenden Ansprüche zur Herstellung eines Arzneimittels zur Induktion einer Tumorimmunität durch Injektion der Hybridzelle in einen an einer B-Zell-Neoplasie erkrankten Patienten, aus welchem die maligne B-Zelle stammt.

## Claims

1. Hybrid cell which can be produced by fusion of a malignant B cell derived from a patient and a hybridoma cell wherein the latter produces an antigen which binds to a surface antigen on an antigen-presenting cell (APC).

2. Hybrid cell according to claim 1,
**characterized in that**
said malignant B cell is a B cell leukemia cell, a B cell lymphoma cell, or a plasmocytoma cell.

3. Hybrid cell according to claim 1,
**characterized in that**
said hybridoma cell is obtained by the fusion of a lymphocyte producing an antibody against a surface antigen on an APC to a myeloma cell.

4. Hybrid cell according to claim 1,
**characterized in that**
said malignant B cell produces immunoglobulins which assemble with the immunoglobulin of the antibody-producing hybridoma cell to form bispecific antibodies.

5. Hybrid cell according to claim 4,
**characterized in that**
the bispecific antibody is secreted into the surrounding medium.

6. Hybrid cell according to claim 4 or 5,
**characterized in that**
the bispecific antibody binds to Fc receptors.

7. Hybrid cell according to claim 4 or 5,
**characterized in that**
the bispecific antibody binds to mannose-5 receptors.

8. Hybrid cell according to claim 4 or 5,
**characterized in that**
the bispecific antibody binds to MHC class II antigens.

9. Hybrid cell according to claim 1,
**characterized in that**
said hybrid cell is not rendered replication incompetent prior to injection into the patient.

10. Hybrid cell according to claim 1,
**characterized in that**
said hybrid cell is rendered replication incompetent by irradiation prior to injection into the patient.

11. Hybrid cell according to claim 1,
**characterized in that**
said surface antigen on the antigen-presenting cell is a Fc receptor, a mannose-5 receptor, or a MHC class II antigen.

12. Pharmaceutical composition,
**characterized in that**
said pharmaceutical composition contains a hybrid cell according to claim 1 in a therapeutically effective amount together with one or more pharmaceutically acceptable carriers and/or excipients. acceptable carriers and/or excipients.

13. Process for the preparation of a hybrid cell according to claim 1,
**characterized in that**
a malignant B cell derived from a patient is fused to a hybridoma cell producing an antibody which binds to a surface antigen on an antigen-presenting cell (APC) by a process known per se.

14. Use of a hybrid cell according to claim 1 for the induction of tumour immunity by injecting the hybrid cell into a patient suffering from B cell neoplasia from whom the malignant B cell is derived.

## Revendications

1. Cellule hybride, pouvant être fabriquée par fusion d'une cellule maligne B provenant d'un patient et d'une cellule d'hybridome, qui produit un anticorps qui se lie à un antigène de surface sur une cellule présentant des antigènes (APC).

2. Cellule hydride selon la revendication 1, **caractérisé en ce que** la cellule maligne Z est une cellule leucémique B, un lymphome d'immunotype B ou une cellule plasmocytaire.

3. Cellule hydride selon la revendication 1, **caractérisé en ce que** la cellule d'hybridome résulte de la fusion d'un lymphocyte, qui produit un anticorps contre un antigène de surface sur une APC, et un myélocyte.

4. Cellule hydride selon l'une quelconque ou plusieurs des revendications précédentes, **caractérisée en ce que** la cellule B produit de l'immunoglobuline qui s'assemble avec l'immunoglobuline de la cellule d'hydridome produisant les anticorps pour former des anticorps bispécifiques.

5. Cellule hydride selon l'une quelconque ou plusieurs des revendications précédentes, **caractérisée en ce que** l'anticorps bispécifique est sécrété dans le milieu environnant.

6. Cellule hydride selon l'une quelconque ou plusieurs des revendications précédentes, **caractérisée en ce que** l'anticorps bispécifique se lie à des récepteurs FC.

7. Cellule hydride selon l'une quelconque ou plusieurs des revendications précédentes, **caractérisée en ce que** l'anticorps bispécifique se lie à des récepteurs de mannose-5.

8. Cellule hydride selon l'une quelconque ou plusieurs des revendications précédentes, **caractérisée en ce que** l'anticorps bispécifique se lie à des antigènes de classe II MHC.

9. Cellule hydride selon l'une quelconque ou plusieurs des revendications précédentes, **caractérisée en ce qu'**elle n'est pas rendue incapable de réplication avant l'injection dans le patient.

10. Cellule hydride selon l'une quelconque ou plusieurs des revendications précédentes, **caractérisée en ce qu'**elle est rendue incapable de réplication par irradiation avant l'injection dans le patient.

11. Cellule hydride selon l'une quelconque ou plusieurs des revendications précédentes, **caractérisée en ce que** l'antigène de surface sur la cellule présentant des antigènes est un récepteur Fc, un récepteur de mannose-5 ou un antigène de classe II MHC.

12. Composition pharmaceutique, **caractérisée en ce qu'**elle contient une cellule hydride selon l'une quelconque ou plusieurs des revendications précédentes selon une quantité thérapeutiquement active avec un ou plusieurs porteurs et/ou adjuvants pharmaceutiquement compatibles.

13. Procédé de fabrication d'une cellule hydride selon l'une quelconque ou plusieurs des revendications précédentes, **caractérisé en ce que** l'on fusionne une cellule maligne B provenant d'un patient avec une cellule d'hybridome, qui produit un anticorps qui se lie à un antigène de surface sur une cellule présentant des antigènes (APC) à l'aide d'un procédé connu en soi.

14. Utilisation d'une cellule hydride selon l'une quelconque ou plusieurs des revendications précédentes pour la fabrication d'un médicament pour induire une réponse immune contre une tumeur par l'injection de la cellule hybride dans un patient souffrant d'une néoplasie de cellule B dont provient la cellule maligne B.
